# EUROPEAN PATENT APPLICATION

(11) **EP 4 781 810 A1**
(43) Date of publication of application: **29.07.2026**
(21) Application number: 25154550.5
(22) Date of filing: 28.01.2025
(51) Int. Cl.: A01B 79/00, A01M 7/00, B01L 3/00, C12Q 1/6806, C12Q 1/6844, C12Q 1/6851, C12Q 1/686, C12Q 1/6888, C12Q 1/689, C12Q 1/6895, G01N 1/22, G01N 1/24, G01N 15/06

(54) **DEVICE, SYSTEM AND METHOD FOR PLANT DISEASE DETECTION AND FORECAST**

(71) Applicant: EFOS d.o.o., 6225 Hrusevje (SI)
(72) Inventor: STEFANCIC, Matej, 6225 Hrusevje (SI); GERZELJ, Nika, 6225 Hrusevje (SI); STEFANCIC, Mateja, 6225 Hrusevje (SI); BOZIC, Bostjan, 6225 Hrusevje (SI)
(74) Representative: Balder IP Law, S.L.

(57) **Abstract**

A method for predicting diseases in crops, comprising: receiving (501) an air sample (204), capturing (502) particles (205) from the received air sample (204), performing (503) a DNA amplification reaction on the captured particles (205), extracting (504) at least one attribute of the reaction, said at least one attribute representing a presence/absence and/or abundance of an airborne pathogen that may be comprised in the captured particles (205), acquiring (505) weather data, wirelessly sending (506) the at least one attribute of the reaction and the acquired weather data to remote computing means comprising a prediction model, periodically repeating the former steps, and at the prediction model of the remote computing means, making a prediction (507) of airborne pathogen-based crop disease presence or development, said prediction being based at least on the at least one attribute of the reaction and the acquired weather data. System and device.

## Description

### TECHNICAL FIELD

The present invention relates to the field of pest disease monitoring and control. More particularly, it refers to devices, methods and systems for monitoring pests created by airborne pathogens and/or for performing a pest forecast based on the monitoring.

### BACKGROUND

Monitoring and predicting development of airborne diseases is the basis of all modern technologies of agricultural production. Most of the prediction of the development of this kind of diseases is done based on weather data where parameters like temperature, leaf wetness, relative humidity and precipitation play a major role. However, weather data is usually not enough to make accurate predictions, since it only indicates favourable conditions for the development of diseases. To predict the effect a disease will have on the crops, it is crucial to understand whether spores of a given disease are present and active. Collecting such information is typically associated with manual data collection with spore catchers in the field and time-consuming analysis like preparing samples to be analysed in a laboratory.

In order to be efficient, contemporary agricultural techniques require intensive control and monitoring of diseases that may affect crops. In the past, methods for monitoring and predicting plant diseases have been developed due to the growing demand for better crop protection.

New crop protection products must comply with increasingly stricter food safety regulations. Said regulations demand to apply crop protection in the right time frame to eliminate a pathogen when it becomes active and before the symptoms of the disease cause damage on crops. Pesticides, such as fungicides and bactericides, are used in large quantities when it is predicted that a disease may occur. Such use of pesticides usually leads to development of resistance of pathogens to the products used.

In this sense, evaluation of environmental fungal bioaerosols using spore trap samplers has become a profitable industry (Robertson and Brandys, 2011). The vast majority of devices for monitoring airborne plant diseases are not automated and require manual work and special laboratory equipment or are semi-automated, but do not provide real time information about development of disease in the field. This data is crucial for optimal timing of applying crop protection.

As air borne diseases are caused primarily by fungi and bacteria, they are hard to detect and monitor before the symptoms appear on plants and crop yield. Spores of fungi and bacteria that are caught on sampling devices are transferred to laboratories where technicians examine samples by analysis means, like optical microscopy. The analyses of spores are often prone to subjective decisions based on the skill and experience of the technician carrying the analysis, as detailed in "Robertson and Brandys, 2011, DOI: 10.3852/10-017". Considering the above, analyses that are based on detecting the DNA of the spores are more accurate and reliable than optical analyses, allowing a potential monitorization of the analysis process.

As a matter of example, U.S. patent application US2022307967A1 discloses a spore trap device for detecting the presence of spores in crop leaves comprising optical detectors such as photomultipliers or photodiodes, for example avalanche photodiodes. U.S. patent application US 2022/349786 A1 describes real-time acquiring and detecting airborne fungus particles collected from the air, comprising a camera for collecting images of fluorescent data of the collected fungi. Furthermore, international patent application WO2020128028A1 discloses a method for detecting Botrytis cinerea fungal spores based on DNA extraction. However, the drawback of these disclosures is the necessity of checking the device in the field for accessing the measured data.

Automation of airborne pathogens sample collection, DNA extraction and data collection are valuable tools for the monitoring of diseases in crops. International patent application WO2022232510A1 discloses an automated detection system of air borne plant diseases based on DNA extraction. In this case, results data are remotely accessible, for example in the form of alerts, however the system still requires a manual sample preparation to be performed in the field.

The prediction of possible diseases caused by airborne pathogens is needed to anticipate any action required on the crops, once airborne pathogens are detected. The early detection of airborne pathogens allows for example to optimize the process of fumigating or spraying of a crop field, minimizing a potential crop damage.

In view of the above, there is a need for a device, method and system for automatically and timely detecting airborne pathogens, such as fungus spores, bacteria or viruses on crops. There is also a need for a method and system for forecasting the development of diseases caused by said airborne pathogens, which enables to minimize crop losses and optimizing the fumigating of crops and other control measures.

### DESCRIPTION OF THE INVENTION

The proposal for detection of airborne pathogens and prediction of airborne pathogen-based diseases in crops described in the present disclosure overcomes the drawbacks of prior art techniques therefor.

The present disclosure provides a method and system for making predictions of the presence/absence and/or development of airborne pathogen-based diseases in crops.

The present disclosure also provides a device for automatic airborne pathogen detection. The device may be used by the method and system for making predictions of the presence/absence and/or development of airborne pathogen-based diseases in crops.

In the context of the present invention, and as is well known in the field, airborne crop pathogens (also referred to simply as airborne pathogens) are microorganisms that cause diseases in plants and are transmitted through the air. These pathogens may include, among others, fungi, bacteria, or viruses.

The airborne pathogens may spread without a transport means or medium, such as free floating in the air or dispersed in the air in individual pathogen cells or microorganisms. The pathogens may be free floating in the air making agglomerates of pathogens, each agglomerate comprising more than one individual pathogen cell or microorganism. Said agglomerates may comprise more than one type of airborne pathogen. The airborne pathogens may also travel in the air contained in a transport means or medium, such as aerosols. The aerosol may be a speckle of dust or fluid droplet carrying one or more individual pathogen cells or microorganisms. Said aerosol may comprise more than one type of airborne pathogen.

In the context of the invention, the expression "particles" is used to refer to any aerosol, speckle of dust, droplet or the like that may contain or not contain an airborne pathogen, as well as to any single free floating airborne pathogens and to free floating airborne pathogens forming agglomerates.

A first aspect of the disclosure relates to a system for predicting diseases in crops. The system comprises:
at least one device for airborne pathogen detection, the at least one device comprising:
   means for receiving an air sample,
   means for capturing particles from the received air sample,
   means for performing a DNA amplification reaction on the captured particles,
   means for extracting at least one attribute of the reaction, said at least one attribute representing a presence/absence and/or abundance of an airborne pathogen that may be comprised in the captured particles, and
   a transceiver for wirelessly transmitting the at least one extracted attribute of
the reaction; and
remote computing means comprising a prediction model, the prediction model being configured to make a prediction of an airborne pathogen-based crop disease presence or development based at least on the at least one extracted attribute of the reaction and the acquired weather data.

The remote computing means may be located in the cloud. For example, it may be a cloud server or may belong to a cloud server. The remote computing means may be a remote station. The remote computing means may include equipment in different locations, such as local equipment (for example a local server) and cloud equipment (for example a cloud server). The remote station may be located at a distance from the crop field.

The remote station is preferably configured to wirelessly receive the at least one extracted attribute of the reaction, which is sent by the transceiver of the at least one device, and the acquired weather data, which may be acquired by the at least one device or by weather stations disposed, for example, in a crop field. Therefore, in some embodiments, the at least one device further comprises means for acquiring weather data.

The at least one device is configured to be placed in a crop field or in any location in which it might be desirable to detect the presence and/or development of airborne pathogens.

The at least one attribute may represent presence/absence and/or abundance of more than one airborne pathogen that may be comprised in the captured particles.

If no pathogen is detected in the sample, it can be assumed that the pathogen is not present or that it is present in a sufficiently low amount to conclude that it does not pose threat to a crop.

The prediction model can identify complex patterns and correlations between detected airborne pathogens, weather conditions, and/or crop disease development or symptoms, allowing for precise prediction of diseases development in the crop field. The prediction model can differentiate between diseases that may be caused by diseases having similar symptoms or caused by similar pathogens, thus reducing false positives or misidentifications of diseases.

To perform a prediction, the remote computing means takes into account the particular location (a particular device when there is more than one device) to which the received data belong. In other words, the remote computing means knows the coordinates of the point, for example in a crop field, in which certain attributes and weather data have been acquired. The remote computing means also takes into account the acquisition time of the received data.

The at least one device may further comprise means for breaking a cell wall of the airborne pathogen that may be comprised in the captured particle for liberating DNA of the airborne pathogen prior to performing the DNA amplification reaction. Said means may be, for example, mechanical means, chemical means or means involving application of radiation. Preferably, means involving application of radiation are used, such as a microwave generator (heat application) or ultrasound waves generator. The use of such techniques reduces the complexity of the device compared to a device requiring mechanical means (such as grinding) directly actioning on the cell wall for breaking the cell wall while does not require chemicals/enzymes methods.

In some embodiments, the means for performing a DNA amplification reaction is configured for performing Loop-Mediated Isothermal Amplification (LAMP). Alternatively, the means for performing DNA amplification may perform other DNA amplification techniques, such as q-PCR or RT-PCR. Said DNA amplification reactions can identify very low concentrations of pathogens potentially present in the analysed particles, allowing a precise detection of pathogens.

Good diagnostic test must comprise sensitivity, specificity, low-cost, simplicity, rapidity and adaptability to all kinds of climatic changes and to the availability of instruments. Among the DNA amplification techniques, LAMP stands out for its rapidity, sensitivity and specificity. Unlike other DNA amplification techniques, LAMP can be performed in several minutes, since initial heat denaturation for DNA template is not required. Additionally, the LAMP method needs lower temperature to perform amplification compared to other methods. The analysis of the results is also simple when the LAMP method is used. Direct evaluation of results is possible as the dyes used in LAMP reactions usually enable naked eye-observation (or camera operating in visual spectrum) of colorimetric changes either under natural light or UV light.

Preferably, the acquired weather data comprises information of at least one of the following weather parameters: temperature, relative humidity and precipitation. More preferably, the acquired weather data comprises information of the following parameters: temperature, relative humidity and precipitation, and optionally of at least one of: leaf wetness, wind direction, wind gust and wind speed.

The at least one device may comprise means for acquiring weather data. The means for acquiring weather data may be configured to measure and to provide to the transceiver for subsequent wirelessly transmission to the remote computing means, the following weather parameters: temperature, relative humidity and precipitation. It optionally measures and provides at least one of the following weather parameters: leaf wetness, wind direction, wind gust and wind speed. The means for acquiring weather data is for example a weather station. They are acquired with suitable sensors with which the means for acquiring weather data may be equipped. Other weather parameters may additionally be acquired, such as wind direction and/or wind speed, contributing to characterize the atmospheric conditions around the crops.

The means for acquiring weather data with which the device may be equipped comprises conventional weather sensors.

Alternatively, weather data may be acquired from crop close-by weather stations or localized weather forecast. Said acquired weather data may be wirelessly transmitted to the remote computing means. The same or similar weather parameters as those acquired by means comprised in the at least one device, may be acquired by crop close-by weather stations or localized weather forecast.

The captured weather data provided to the remote computing means is used by the prediction model to make a prediction of an airborne pathogen-based crop disease presence or development.

In embodiments, the means for capturing particles from the received air sample is a catching means configured, in use of the device, to catch at least one particle potentially present in the air sample entering the device. The catching means may be, for example, a suitable paper, such as quartz paper.

The at least one device may comprise rotating means configured to rotate the means for capturing particles from a first position (such as a particle-catching position) to a second position (such as a particle DNA amplification position). This allows to optimize the catching of particles in the first position and the DNA amplification in the second position. Typically, an initial position of the rotating means positions the means for capturing particles perpendicular to the direction of the air, such that the catching of potential airborne pathogens present in said air sample is maximized. When air samples enter the device through an inlet (window or hollow made on a wall of the device), the means for capturing particles are, in the initial position, parallel to this inlet. The initial position of the catching means may be a vertical position. A final position of the rotation means positions the means for capturing particles parallel to the means for performing the DNA amplification reaction from the received air sample. The means for capturing particles in the final position may be above the means for performing the DNA amplification reaction. The final position of the means for capturing particles may be a horizontal position. The means for capturing particles repeatedly alternates from the initial (or first) position to the final (or second) position, so that the process of catching airborne pathogen and subsequently causing a DNA reaction to extract an attribute of the reaction, is repeated in time, typically periodically. The means for capturing particles may reset its configuration (for example, may clean the catching means or may replace the used catching means with new catching means) after returning to the initial position, to avoid cross-contamination of airborne pathogens captured at different times.

In some embodiments, the at least one device further comprises means for providing reagents and/or primers, for example in the form of a fluid) to the means for capturing particles. The fluid may then be used to perform the DNA amplification reaction. The provided reagents and/or primers are selected to target a particular type, class, order or any other classification definition that define an airborne pathogen. Said primers and/or reagents (or fluids containing them) may target a specific virus, a class of bacteria or any mix of any possible airborne pathogens. The subsequent presence/absence and/or abundance of the attribute extracted from the DNA amplification reaction may denote the presence/absence and/or abundance of the particular pathogen that the primer is targeting. The reagents and/or primers may be in solid or fluid (preferably liquid) state. Preferably, reagents and/or primers are in liquid state, being a means for samples to reach the means for performing a DNA amplification reaction and to allow heating up of the sample containing the airborne pathogens.

In some embodiments, the means for performing said DNA amplification reaction in the at least one device is a microfluidic chip. In the microfluidic chip, the DNA amplification reaction is performed and a signal, for example a fluorescence signal, such as a fluorescence image, based on the amplified DNA is generated. The signal, such as fluorescence signal, is generated if an airborne pathogen or a particle comprising airborne pathogen is captured and the DNA amplification is performed on said pathogen. When the generated signal is a fluorescence image, the means for extracting the at least one attribute of the DNA amplification reaction is for example a fluorescence detector/sensor, such as a fluorescence camera. More than one attribute may be extracted by the means for extracting the attribute.

The means for acquiring weather data may be, for example, a portable weather station or sensors capable of acquiring the respective data of interest.

In embodiments, the at least one device further comprises a control unit configured to control some or all the elements comprised in the device. For example, the control unit may control the means for capturing particles from the received air sample, the means for performing a DNA amplification reaction on the captured particles, the means for extracting at least one attribute of the reaction, the means for acquiring weather data (if the device is equipped with this means), and/or the transceiver. This control unit allows to centralize the control of the device for a coordinated and precise functioning of the different elements in the device, thus enabling automatic detection of airborne pathogens. For example, by controlling the transceiver, the control unit is capable of controlling the sending and receiving of data to the remote computing means.

In embodiments in which the at least one device includes rotating means configured to rotate the means for capturing particles, the control unit controls this rotating means, the means for providing a fluid and the microfluidic chip, such that the position of the rotating means is alternatively changed from a first position, to optimize the amount of particles to be captured in the means for capturing particles, to the second position, to optimize the provision of fluid to the means for capturing particles, and vice versa. In other words, the means for capturing particles is automatically moved from one initial position to the final one, thus enabling the automatic optimization of the airborne pathogen detection process. Additionally, the control unit may control the means for capturing particles such that a new clean catching means is exposed for acquiring new particles to avoid cross contamination between particles in air samples caught at different times. In particular, the control unit may control the unrolling of a first spool comprising new (not yet used) catching means, and the rolling of a second spool comprising used catching means.

In some embodiments, the catching means comprises a first spool of particle catching element, a second spool of particle catching element, and a section of the particle catching element extending between the first spool and the second spool, said section configured for catching airborne particles contained in the air sample.

In some embodiments, the catching means further comprising means for rolling up or down the first spool around a first axis and means for rolling down or up the second spool around a second axis, wherein in the means for rolling up or down the first spool and the means for rolling down or up the second spool are configured to roll in a same direction, enabling exposure of a new section of particle catching element to extend between the first spool and the second spool.

In preferred embodiments, the system comprises a plurality of devices, each device being configured to be placed at a different location in the, for example, crop field in which the system is deployed. Using a plurality of devices allows to monitor the presence and/or abundance of airborne pathogens more precisely, acquiring data from different parts of a large crop field and collectively covering a large area, ultimately providing more precise data to the prediction model and improving the predicting of diseases. The devices comprise means for registering their respective geographical location, such as GPS or the like, such that the data (DNA amplification attribute data and optionally weather data) provided to the remote computing means is related to the position of the respective device within the crop field. The registered geographical location of a device can be sent to the remote computing means together with the data (DNA amplification attribute data and optionally weather data).

Advantageously, the automatization of the DNA amplification reaction, the acquisition of weather data and the prediction generated by the prediction model cause a reduction of human errors possibly leading to a misidentification of pathogen and its impact on the crop.

The prediction of an airborne pathogen-based crop disease presence or development based at least on the at least one extracted attribute of the reaction and on acquired weather data is provided for a given time horizon, such as for the next N days. For example, N can be 7 days, or 10 days, or 14 days.

To make a prediction of an airborne pathogen-based crop disease presence or development, the prediction model may use, in addition to the at least one extracted attribute of the DNA amplification and the weather data, known correlations about how airborne pathogen diseases develop under different weather conditions. Hence, the prediction model can provide, for a given weather forecast (for example for the next N days), a prediction of timing and intensity of different stages of an airborne pathogen disease development.

In embodiments, the prediction model comprised in the remote computing means is a machine learning model. Other types of prediction models may be used alternatively or in combination with the machine learning model, such as statistics-based models or neural networks.

The machine learning model has been trained using a sufficiently representative amount of training data. For example, the prediction model is parametrized/trained using training data like correlation of weather data and dynamics of development of airborne pathogen diseases. The training data (training data sets) comprises: captures of airborne pathogen(s); weather data (typically captured from the crop field of interest or from locations within a certain distance from the crop field of interest); and a development pattern for one or more diseases (of the potentially captured airborne pathogen(s)) under certain weather conditions. The captures of airborne pathogen(s) typically comprise information of presence and/or abundance of the potential pathogen(s) to be detected.

Optionally, the training data may also comprise data of a crop phenological state. The crop phenological state can improve the accuracy of the prediction because the phenological state of a crop can be a factor in disease development. The phenological state of the crops may comprise information about the life cycle plant and its status, for example, germination, leaf emergence, flowering or fruiting. Different airborne pathogens may affect the crops depending on the phenological state, which is used during the prediction. The disease development status may comprise past disease evolution of a crop caused by an airborne pathogen under different weather conditions.

Optionally, the training data may also comprise data of applied crop protection actions. Also optionally, the training data sets can include crop variety.

The weather data may comprise frequent (usually periodic) readings of weather data. Preferably, the following weather parameters are comprised in the weather data: temperature, relative humidity and precipitation. Other weather parameters like wind speed, wind gust, wind direction and leaf wetness can optionally be included. For example, weather data may be captured at time intervals of several minutes, such as 15-minutes interval, or 10-minutes interval, or 5-minutes interval. Disease development patterns may vary from disease to disease. They may even vary for a given disease, even under same weather conditions, since disease development can be dependent on crop and on crop variety. Other sorts of training data may also be optionally used.

Optionally, the training data may be from crop fields different from the crop field where the devices are located, provided the different crop fields have similar weather conditions and crops to the crop field where the devices are located. For example, the weather data used in the training of the prediction model may correspond to different geographical locations and may include locations within the crop field for which a prediction is to be made and locations different from the crop field, for example within a distance range such that the weather may affect the crop field within a time period. In embodiments, the training data may be obtained from crop fields different from the crop field where the devices (for the actual prediction) are located, provided that the different crop fields have similar weather conditions and crops to the crop field where the devices are located.

The crop phenological state data optionally used for the training of the model may be data obtained by on-site observation (such as from tractors or sprayers) or data obtained by remote observation (such as with drones or satellite), of the stage of the crop development. In addition, crop protection data which may comprise, for example, spraying of crops, such as type of crop protection product, concentration, time of application or location in the crop field where the fumigation is performed, can optionally also be used in the training of the model to subsequently better predict the impact the pathogen will have on the crop.

Optionally, during training of the machine learning model, statistical models may additionally be used. For example, statistical models may comprise population dynamics parameters, like previous pathogen spores presence/absence and/or abundance, data regarding crop protection and/or disease eradication measures.

The prediction model (in combination with the at least one device) can efficiently and remotely monitor large crop fields and process large amounts of data without requiring a proportional increase in human intervention. In some embodiments, the machine learning model is configured to predict the timing and intensity of disease development of the captured airborne pathogen. For example, it can provide pairs of time versus intensity of a disease development, for a collection of time periods within a certain time horizon (the time horizon being, for example, the next N days).

The prediction model can continuously improve and adapt by learning from new data over time. This can either be due to better input data (like more accurate population dynamics parameters; more accurate weather forecast) or by re-training using additional relevant data sets not available in previous iterations of model training.

In some embodiments, the prediction model may be trained periodically (retrained) using the data from the extracted attribute of the DNA amplification reaction and the measured weather data from the at least one device or by using other relevant data sources not available during previous iteration of model training. This enables more accurate predictions of a disease.

Regarding the actual prediction made by the trained prediction model, in embodiments, a weather forecast may be used by the computing means for the prediction. Said weather forecast may comprise for example 7 to 10 days of weather forecast and may comprise information of some or all of the following parameters: temperature, relative humidity and precipitation. Preferably, all the former parameters are used. Other optional weather parameters to be used are leaf wetness, wind direction, wind gust and/or wind speed.

The prediction model may perform a prediction by using information learnt from historical crop diseases caused by airborne pathogens, weather data and/or patterns and/or crop characteristics, to predict future airborne pathogen crop disease development based on the acquired data. The prediction may be done before the appearance of visible crop disease symptoms, allowing an early detection and minimizing the impact of the crop disease significant crop loss. Said early detection is typically sooner than the detection of a crop disease made with manual systems requiring the intervention of humans. Also, the system may diagnose the present existence of a pathogen present in the crop field, based on the result of the DNA amplification reaction.

The prediction model may generate a new prediction any time a new value in a periodically extracted attribute (pathogen presence analysis) or weather data is measured. Alternatively, the prediction model may re-run a prediction periodically, such as every hour, every 6 hours or every day, independently of the results of the DNA amplification reaction or the weather data. The DNA amplification reaction and/or the weather data acquisition may be periodically repeated independently of the resulting measurement, such as for example, every 15 minutes, every hour, every 6 hours or, preferably, every day.

Non-limiting examples of the periodicity with which the steps of the method are periodically repeated are: 1 minute, 1 hour, 6 hours or 1 day. The periodicity may be increased or decreased accordingly depending on the time evolution of the DNA amplification reaction or the measured weather data.

In some embodiments, an action is performed based on the prediction generated by the prediction model.

In embodiments, the prediction may include an indication or recommendation to apply crop protection. The indication or recommendation to apply crop protection may comprise information about the type of fungicide, the concentration, the location to be applied or similar. The indication to apply crop protection may depend on the prediction made by the prediction model, in particular if the prediction model predicts a risk of a development of a disease produced by the airborne pathogen which can be remedied with a fungicide or other disease treatment agent. Based on the indication or recommendation, crop protection is applied on the crop field. For example, the applied crop protection may be spraying of the crops with a fungicide or other relevant crop protection product.

In another example, based on the prediction, an alert alerting of the potential presence or development of a disease may be generated. The remote computing means may generate an alert based on the prediction generated by the prediction model. The alert may take different forms: the alert may be a binary alert, i.e. indicating the presence or absence of disease or of risk of disease; or the alert may be a numerical alert indicating a probability of presence or absence of disease or of risk of disease; or the alert may comprise a text informing about the details of the predicted crop disease, such as for example, time of the start of the disease, intensity of the disease or required actions to take on the crop field. Said alert may be wirelessly transmitted to an electronic device, such as a mobile phone.

In embodiments, the prediction model embedded in remote computing means is further configured for receiving historic weather data and/or historic or crop disease treatment data. The prediction model uses this historic weather data and/or historic or crop disease treatment data to make a prediction of an airborne pathogen-based crop disease presence or development.

A second aspect of the disclosure relates to a method for predicting diseases in crops, comprising:
receiving an air sample,
capturing particles from the received air sample,
performing a DNA amplification reaction on the captured particles,
extracting at least one attribute of the reaction, said at least one attribute representing a presence/absence and/or abundance of an airborne pathogen that may be comprised in the captured particles,
acquiring weather data,
wirelessly sending the at least one attribute of the reaction and the acquired weather data to a remote computing means comprising a prediction model,
periodically repeating the previous steps, and
at the prediction model of the remote computing means, making a prediction of an airborne pathogen-based crop disease presence or development,
said prediction being based at least on the at least one attribute of the reaction and the acquired weather data.

The steps of receiving an air sample, capturing particles from the received air sample, performing a DNA amplification reaction on the captured particles, extracting at least one attribute of the reaction, said at least one attribute representing a presence/absence and/or abundance of an airborne pathogen that may be comprised in the captured particles, and wirelessly sending the at least one attribute of the reaction to a remote station, are performed in at least one device for airborne pathogen detection.

The step of acquiring weather data may be performed by the at least one device for airborne pathogen detection or by a different device, such as a weather station located within a certain proximity of the at least one device.

Advantageously, the method allows to automatically and continuously monitor the presence of pathogens.

The method reduces the need for constant human intervention. The method allows continuous surveillance of presence and abundance of airborne pathogen. Therefore, the necessity of periodic manual measurement or observations is eliminated or at least drastically reduced. All this can result in an improved data collection, especially in large crop fields or in smaller fields scattered over larger area where the monitoring can be labour intensive.

The received air sample typically comprises air from the surroundings of the device placed for example in the crop field.

The potential airborne pathogen captured from the air sample may be a microorganism such as fungi, bacteria or viruses, or a combination thereof.

In embodiments, the DNA amplification reaction is Loop-Mediated Isothermal Amplification (LAMP). Other types of DNA amplification techniques may be used, such as q-PCR, rt-PCT (quantitative Polymerase Chain Reaction) or RPA (Recombinase Polymerase Amplification).

In some embodiments, the DNA amplification reaction generates a fluorescence signal based on the amplified DNA. Other attributes of the DNA amplification reaction may be generated and/or extracted, such as colorimetric detection, lateral flow assay, electrochemical detection or temperature of the reaction. The DNA amplification reaction may generate more than one fluorescence signal, wherein each signal may represent the amplified DNA of a different pathogen. Fluorescence emission is typically correlated to the presence and quantity of a targeted DNA. The DNA amplification reaction may generate a fluorescence signal even when the received air sample does not comprise particles, hence, air sample not comprising an airborne pathogen. Said generated fluorescence signal may not generate a significantly measurable fluorescence signal, meaning that no amplification has taken place in the DNA amplification reaction. The lack of fluorescence signal implies that the particularly targeted airborne pathogen has not been captured by the device. Hence, the extracted attribute may be equal to zero, meaning that the DNA amplification reaction does not amplify any DNA due to the absence of the particularly targeted airborne pathogen. Other airborne pathogens that were not targeted during the DNA amplification may be present in a reaction that generates no fluorescence signal. A fluorescence sensor may take fluorescence readings every few seconds. With such time series of fluorescence readings, a graph representing how quickly and how intensive the fluorescent reaction was, can be derived. Thus, presence and quantity of a targeted pathogen DNA is obtained.

In some embodiments, attribute data measured from the result of the DNA amplification technique may be, for example, an amount of fluorescent light measured and/or an intensity of the reaction at different time snapshots. In some embodiments, the fluorescence light emission is correlated to the presence and quantity of the amplified DNA. A fluorescence sensor, such as a camera or a sensor capable of measuring fluorescence light, may take fluorescence reading periodically, such as every 10 seconds. Then, from the periodic fluorescence readings, the attribute data showing the appearance and intensity of the fluorescent light can be derived, hence, describing the presence and quantity of targeted pathogen amplified DNA.

In some embodiments, the temperature to which the sample has been heated may also be measured. A time associated to the measurement is preferably measured. Said time may be the duration of the fluorescent light or a time stamp of the measurement. The extracted attribute may comprise an amount of captured fluorescence light and/or a wavelength of said captured fluorescence light. The extracted attribute may also comprise the measured time. The attribute may define the flux of light emitted per unit of time and as a function of the emitted wavelength. The attribute may be extracted at different times during the reaction, generating a series of time snapshots of the result of the reaction.

The periodically extracted attribute may reflect a spore presence and abundance and its time evolution during a certain time period, such as daily.

The disclosure of the acquired weather data made in the first aspect applies to the acquired weather data of the second aspect.

In embodiments, a timestamp is associated to at least some of: start and end of the performed DNA amplification reaction, extraction of an attribute and acquisition of weather data. The data of timestamp associated to those steps or acquired data may be transmitted to the computing means, typically as metadata associated to the attribute and weather data, in order to have a time-coherent set of data. The data of timestamp may be used for generating a correct prediction of a disease development. Said timestamp may be generated or supervised by a control unit located at the device.

In some embodiments, the prediction model is a machine learning model. Other types of prediction models may be used, alternatively or in combination with the machine learning model, such as statistics-based models or neural networks.

As explained in the first aspect, the prediction model has been trained using suitable training data. The disclosure of the prediction model, as well as the training of the prediction model, made in the first aspect, is fully applicable to the prediction model and to the training of the prediction model of the second aspect.

The description of the different components of the devices for airborne pathogen detection disclosed in the first aspect are also applicable to the method of the second aspect, and vice versa. Besides, the description of the prediction model and its training disclosed in the first aspect are also application to the method of the second aspect, and vice versa.

A third aspect of the disclosure relates to a device for automatic airborne pathogen detection, comprising:
an air sample inlet for allowing entry of an air sample into the device,
a means for capturing particles configured to, in use of the device, capture particles from the received air sample, said means for capturing particles being disposed in a first position,
rotating means configured to rotate the means for capturing particles to a second position,
means for providing at least one reactive element to the means for capturing particles when the means for capturing particles is in the second position,
a microfluidic chip configured to perform a DNA amplification reaction on the captured particles (if particles have been captured) and for generating a fluorescence signal based on the amplified DNA of the at least one airborne pathogen that may be comprised in the captured particles,
means for detecting the DNA amplification reaction performed in the microfluidic chip,
means for extracting from the detected DNA amplification at least one attribute of the DNA amplification, said at least one attribute representing the presence/absence and/or abundance of a captured airborne pathogen,
a transceiver configured to wirelessly send said at least one attribute, and
a control unit configured to control the rotating means, the means for providing a fluid and the microfluidic chip, such that the position of the rotating means is alternatively changed from the first position, to optimize the amount of particles to be captured in the means for capturing particles, to the second position, to optimize the provision of fluid to the means for capturing particles, and vice versa.

The particles captured by the means for capturing particles can be pathogens or can comprise pathogens. In this context, when the particles are pathogens or comprise pathogens, the terms "pathogen" and "particle" are used interchangeably. Since pathogens are airborne, if no pathogen is caught by the means for capturing particles, it means that there is high probability that the pathogen is not present in the air sample or that it is present in such small quantities that the pathogen would not cause infestation.

The first position in which the means for capturing particles is disposed to favour the capture of particles from the received air sample, is a position defining a plane substantially perpendicular to the air sample flow direction, so that the air sample reaches or collides against the means for capturing particles in a direction substantially perpendicular to the said means for capturing particles. This way, the capture of pathogens by the means for capturing particles is optimized, since the amount of air leaving the device without colliding against the catching media is minimized.

The rotating means can be any mechanism which permits to move the means for capturing particles from the first position to a second position. The mechanism can be any mechanical or electromechanical mechanism. For example, it can be a mechanism actuated by a motor. The second position is a position which favours the provision of fluid to the means for capturing particles, to subsequently favour the DNA amplification reaction. The provision of fluid is preferably optimized when the second position is horizontal, so that received fluid can spread onto the means for capturing particles. Besides, in this position of operation, the microfluidic chip preferably remains parallel to the catching means, in particular the microfluidic chip is preferably disposed beneath the means for capturing particles, parallel thereto, so that pathogens impregnated with fluid can cause or suffer the reaction.

In use of the device, the means for capturing particles is alternatively moved from the first position to the second position, repeatedly. For example, the device is programmed or configured to have the means for capturing particles in the first position for a certain period of time (such as 15 minutes, or 30 minutes, or 1 hour, or longer) and to, after expiration of that period of time, move the means for capturing particles to the second position, in which it remains for a period of time enough to provide the fluid, perform the DNA amplification reaction, detect the reaction and extract one or more attributes of the DNA amplification. After completion of these operations, the means for capturing particles returns to the first position, in which it remains for the certain period of time, to start a new cycle of capture of pathogens.

In embodiments, the control unit is also configured to control the detection of DNA amplification and the attribute extraction.

The fluorescence signal of the DNA amplification reaction is a light signal generated by the DNA amplification reaction and which indicates the outcome of the reaction. A DNA amplification reaction occurs when one or more pathogens have been captured.

The device can operate automatically, without the necessity of human intervention while the device is in use. The movement of the means for capturing particles from the first position to the second position and vice versa, the start and end of fluid provision towards the means for capturing particles when the means for capturing particles is in the second position, and the detection of DNA amplification and attributes extraction is achieved by the control unit, which coordinates the synchronized operation of the involved elements. The control unit may be, for example, a microcontroller monitoring the coordinated operation of the device in order to perform the sample catching, the DNA amplification, the attribute extraction and the transmission of the attribute to the remote station. Hence, the device enables full automatization of spore catching and detection and transmission, for example to a remote computing means, for further processing, analysis, disease diagnosis and/or disease prediction.

In embodiments, the device further comprises an air pump configured for directing air flow (i.e. the air sample) into the device through the inlet.

In embodiments, the inlet of the device is positioned towards a flow direction of the air sample to enhance the entrance of air on the device. For example, a window of the device may be made perpendicular to a wind direction transporting airborne pathogens, such that the probability of capturing said pathogens is enhanced by facilitating the entry of air sample through the inlet.

In some embodiments, the means for capturing particles comprises a first spool of particle catching element, a second spool of particle catching element, and a section or portion of the particle catching element extending between the first spool and the second spool, said section configured for catching airborne particles contained in the air sample. The particle catching element is preferably a film suitable for catching pathogens. Non-limiting examples of particle catching elements are suitable paper, such as quartz paper.

In some embodiments, the means for capturing particles further comprises means for rolling up or down (that is to say, rolling or unrolling) the first spool around a first axis and means for rolling down or up (that is to say, unrolling or rolling) the second spool around a second axis. The means for rolling up or down the first spool and the means for rolling down or up the second spool are configured to roll in a same direction, enabling exposure of a new, clean section of particle catching element to extend between the first spool and the second spool. The means for rolling up/down the spools may be, for example, an electrical motor to which each spool is attached to. The rolling and unrolling of particle catching element is also controlled by the control unit, in orchestration with the control of other elements of the device, so that automatic operation of the device is achieved.

The first position of the catching means may be substantially perpendicular to the air sample flow, such that the incoming air hit perpendicularly the means for capturing particles (in particular, the portion thereof exposed between the two spools) and thus maximizing the catching probability of a potential airborne pathogen. In other words, when the air inlet (for example, window or hollow made on a wall of the device) is for example disposed on a side wall of the device, when the means for capturing particles is in the first position, the means for capturing particles (in particular, the portion thereof exposed between the spools) is substantially perpendicular to the surface defined by the window or hollow on the wall of the device. The air sample inlet (window or hollow) is therefore preferably perpendicular to the flow of wind.

In the second position, the means for capturing particles s may be in a position substantially parallel to the microfluidic chip. Besides, the catching means and the microfluidic chip are preferably adjacent, meaning that they are in proximity or in contact, such that there is no substantial gap between a surface of the means for capturing particles and a surface of the microfluidic chip. More particularly, the microfluidic chip is preferably parallel to, and beneath, the portion of means for capturing particles onto which particles may have been adhered, such that upon reception of fluid by the means for capturing particles, fluid droplets that may contain pathogens fall on the microfluidic chip, where they can be caused to have a DNA amplification reaction. For example, when the fluid impregnates the catching element, such as quartz paper, portions of the means for capturing particles (that may comprise pathogens) are dissolved and droplets of fluid mixed with dissolved catching media fall due to gravity on specific areas of the microfluidic chip disposed underneath. The microfluidic chip is designed to transport the droplets to different locations of the chip, in which reagents (one or more than one) are added or have been added.

In some embodiments, a reactive element may be automatically added to the particle catching means when the catching means is in a horizontal position. The reactive element may be in the form of a fluid. For example, a dissolvent, such as water, may have the reactive element.

The addition of the fluid may be achieved by releasing drops of fluid by a fluid provider controlled by the control unit. For example, the provision of fluid may be made by mechanical means to ensure proper dosage of reagents to the microfluidic chip chambers. Said fluid may dissolve the particle catching means, or a portion thereof, after a period of time. The control unit is preferably configured to order or trigger the delivery of fluid when the catching means is disposed in the horizontal position.

Subsequently, after the process of DNA amplification reaction, detection and attribute extraction ends, the means for capturing particles may be positioned back to the first position (a position perpendicular to the flow of air) and, by rolling/unrolling the catching element, a new and clean portion of particle catching element is exposed. The rolling/unrolling of catching element may be done either before or after moving the means for capturing particles to the first position. Again, this is controlled by the control unit.

At the microfluidic chip, a treatment may be applied to the received fluid (fluid mixed with dissolved portions of catching means, which may comprise pathogens), for breaking cell walls of the airborne pathogens captured by the particle catching means. The treatment may be a non-mechanical treatment. For example, the non-mechanical treatment may be a bombardment with microwaves or the application of a chemical means.

At the microfluidic chip a DNA amplification technique is applied. The DNA amplification technique may be, for example, LAMP, Polymerase Chain Reaction (PCR) or another DNA amplification technique.

The microfluidic chip may comprise a plurality of engraved channels, wherein several airborne pathogens may be treated simultaneously with the DNA amplification technique. Each engraved channel may be configured to treat a different type of pathogen, thus enabling simultaneous reactions to different pathogens. Alternatively, all or some of the engraved channels may be configured to treat a same pathogen, in which case redundant information may be obtained, which may be important to prevent erroneous detection.

The simultaneous treatment of several airborne pathogens may allow to monitor and predict several diseases simultaneously. Moreover, the simultaneous treatment of several airborne pathogens may allow to predict a combined risk of developing one or more diseases on the crops, caused by a combination of airborne pathogens. It is understood by combined risk as the risk of developing one or more diseases caused by the presence of several airborne pathogens, said one or more diseases appearing due to a specific combination of detected airborne pathogens.

The data collected with this device can be provided to a prediction model like the one disclosed in the first and second aspects to make a prediction of airborne pathogen-based crop disease presence or development.

In embodiments, the microfluidic chip may comprise a plurality of microchannels, each microchannel comprising several chambers. For example, the microfluidic chip comprises 8 microchannels, each microchannel capable of detecting a different pathogen. Fluids, such as reagents, may be automatically added to a chamber. A series of temperature cycles may be performed on each chamber potentially comprising the dissolved airborne pathogen. Once the DNA amplification technique is complete, the remaining solution in a chamber is coloured by adding fluorescent dye, allowing the result of the DNA amplification technique to be read. The result may be measured, for example, optically, by a fluorimeter or similar device.

As an alternative to adding fluids, such as reagent, to a chamber, a pre-prepared chip may be used comprising a dye pre-packed reagent that may be activated when in contact with the sample or when heated to certain temperature.

In embodiments, the device also includes means for acquiring weather data, which can be subsequently provided to the transceiver, so that they can be sent, for example to a remote station. The disclosure of the acquired weather data made in the first and/or second aspects is applicable here.

In embodiments, the means for detecting the DNA amplification reaction performed in the microfluidic chip is a camera configured to capture an image of the reaction.

The at least one device of the system of the first aspect may be the device of the third aspect in any of its variations.

Additional advantages and features of the disclosure will become apparent from the detailed description that follows and will be particularly pointed out in the appended claims.

### BRIEF DESCRIPTION OF THE DRAWINGS

To complete the description and in order to provide for a better understanding of the invention, a set of drawings is provided. Said drawings form an integral part of the description and illustrate an embodiment of the invention, which should not be interpreted as restricting the scope of the invention, but just as an example of how the invention can be carried out. The drawings comprise the following figures:
Figure 1 schematically shows a deployment in a crop field of several devices for automatic airborne pathogen detection and remote computing means.
Figure 2A schematically shows a system for airborne pathogen detection and prediction of an airborne pathogen-based crop disease according to embodiments of the disclosure.
Figure 2B shows the system of Figure 2A, in which the shown device for airborne pathogen detection has been drawn in more detail.
Figure 3A shows a device for automatic airborne pathogen detection according to embodiments of the disclosure.
Figure 3B shows the device of Figure 3A, in which the position of the means for capturing particles has changed to favour the performing of DNA amplification reaction, according to embodiments of the disclosure.
Figures 4A - 4D show the spools of the means for capturing particles of the device shown in Figures 3A and 3B, having captured air particles, and the process of exposing new portions of means for capturing particles, according to embodiments of the disclosure.
Figure 5 shows a flow chart of a method for predicting diseases in crops, according to embodiments of the disclosure.

### DESCRIPTION OF A WAY OF CARRYING OUT THE INVENTION

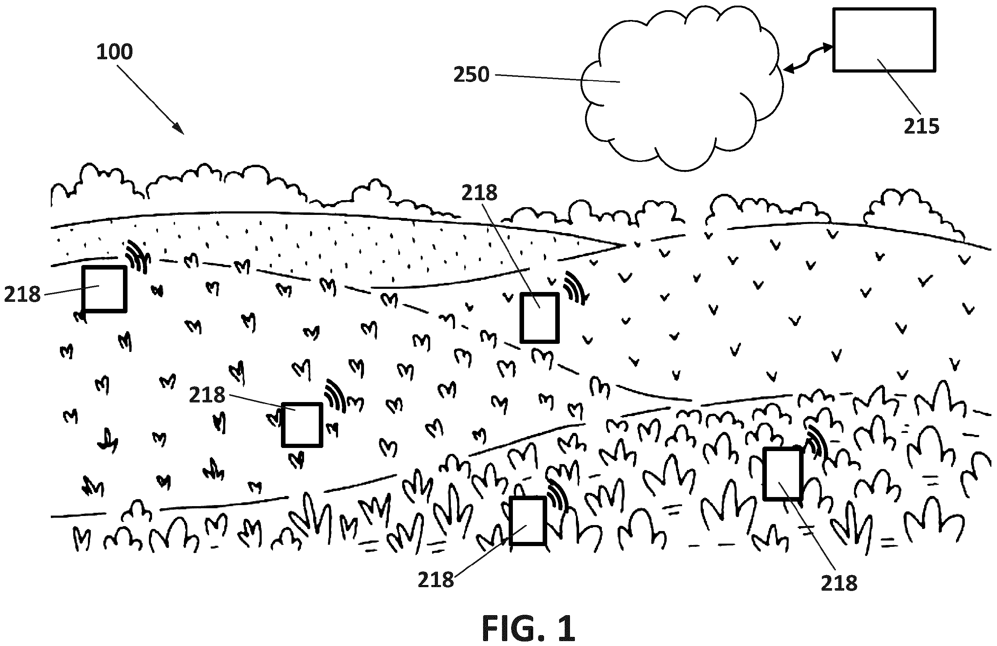

Figure 1 shows an example of a deployment 100 of devices 218 for automatic airborne pathogen detection in a crop field, and a remote unit 215 (for example, a remote computing means, such as a cloud server) for receiving data from the devices and for performing a prediction with the received data. The remote unit 215 is a remote computing means, such as a remote server located, for example, in the cloud. The devices 218 individually perform automatic detection of airborne pathogens from air samples captured from the air surrounding the crop field. Each device 218 communicates with the remote unit 215 through a communication network 250, transmitting the result of the detection. The communication is preferably wireless. The preferably wireless communication is implemented over well-known data communication protocols, such as, but without limitation, cellular communication protocols (such as 3.5G, 4G, 5G or future generations, such as 6G) or short-range communication protocols (such as Wi-Fi, Bluetooth, or the like). The devices 218 may be located at different locations within the crop field. The location of the devices 218 may be substantially equidistant from each other or at a particular distribution, either regular or irregular, according to particularities of the crop field such as, for example, terrain elevation differences or other geographical features, within the crop field. The remote unit 215 may be located at a distance from the crop field smaller than the maximum communication range distance of the furthest device 218. Alternatively, additional communications equipment, such as repeaters, may be used in the event the remote unit is not within the maximum communication range distance.

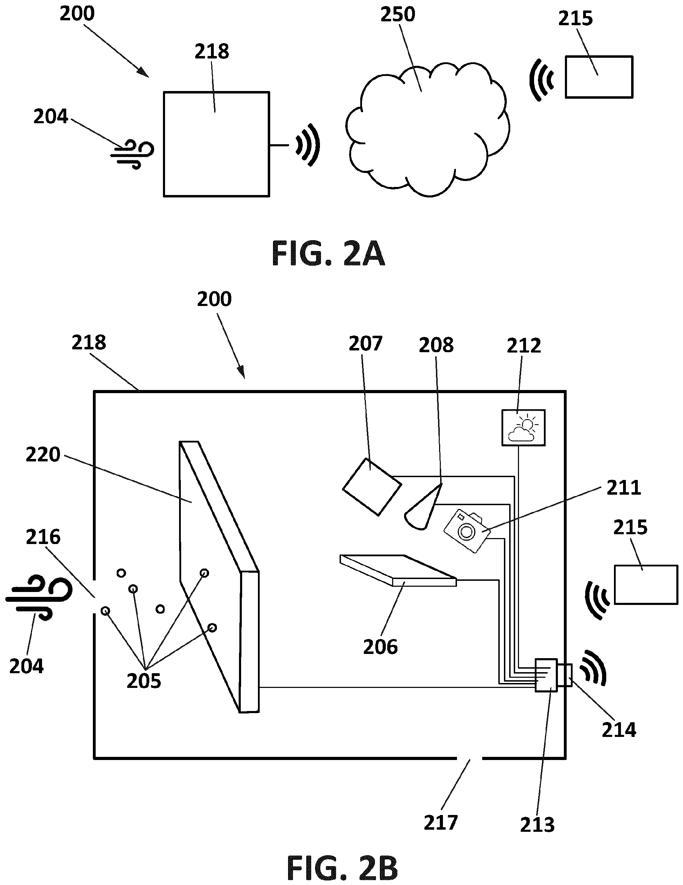

Figure 2A schematically shows a system 200 for predicting diseases in crops according to an embodiment of the present disclosure. For simplicity, a single device 218 for automatic airborne pathogen detection is shown in communication with a remote station 215 for making a prediction of a crop disease development. Generally, such systems 200 typically comprise a plurality of devices 218, intended to be located at different locations in a crop field (as shown in Figure 1), so that a larger area of the crop field is simultaneously monitored. The device 218 receives air samples 204 from the surroundings of the crop field. An air sample 204 may carry airborne pathogens, or particles 205 containing airborne pathogens or water droplets containing airborne pathogens. The device 218 automatically detects airborne pathogens, if present in the sample. The result of the automatic detection is wirelessly sent to the remote station 215.

Figure 2B shows in more detail the device 218 of Figure 2A, or, in general, any of the devices of Figure 1. Air samples 204 enter the device 218 through an air inlet 216 and leave the device 218 through one or more air outlets 217. The air inlet 219 may be a window or hollow made in the wall of the device 218. When the device 218 is deployed in a crop field, the device 218 may be oriented taking into account the wind direction in the selected location. For example, the device 218 may be oriented so that the air inlet 216 is oriented towards a direction of wind (the wind carrying the air sample 204). The device can optionally include a fan, not shown, in the vicinity of the inlet 216, facilitating the entry of air sample 204 into the device 218. When a fan is used, the operation of the fan is preferably stopped once a sample is being processed by the device 218.

The device 218 has a means for capturing particles 220. This means 200 is intended to catch particles 205, potentially containing airborne pathogens, entering the device 218. The catching of particles 205 can be done by any suitable catching means, such as a film allowing the adhesion of airborne pathogens or particles containing airborne pathogens onto said film. The film is for example quartz paper. The detection of airborne pathogens is performed at a microfluidic chip 206. The microfluidic chip 206 is for example a piece of glass having thin channels and small cavities or chambers. By thin and small it is understood a size in the order of magnitude of the size of the airborne pathogens or particles 205 containing airborne pathogens. At the microfluidic chip a DNA amplification reaction on the DNA present in the caught particles 205 (assuming that they contain airborne pathogens) is performed. To enable this reaction, the DNA of the pathogen must be released. In the shown embodiment, this is done by applying a microwave radiation using a microwave source or generator 207. The applied microwave radiation crushes the cell wall of the airborne pathogens, so that DNA is released. The microwave radiation also increases temperature, thus accelerating the reaction. The microwave source 207 is preferably located in the vicinity of the microfluid chip 206, in a location and orientation such that it is able to direct the microwaves at the chambers on the microfluidic chip 206 to heat the fluid and enable the DNA amplification reaction.

To enable the reaction, reagents and/or primers targeting a particular airborne pathogen are required. In the shown embodiment, the necessary reagents and/or primers are provided by a dispenser 208, from which reagents and/or primers are delivered towards the particles/potential pathogens captured on the means for capturing particles 220. The dispenser 208 provides reagents or primers that can be in solid or fluid (preferably liquid) state. Preferably, reagents or primers in liquid state are provided, since liquid reagents or primers also act as a medium for particles/airborne pathogens to reach the chambers of the microfluidic chip 206. Liquid reagents or primers also allow heating up of the sample containing the airborne pathogens.

Once the reaction takes place in the microfluidic chip 206, a sensor 211 measures a resulting attribute of the DNA amplification reaction. In the shown example, the sensor 211 is a camera configured for capturing images of an attribute of the reaction. When the DNA amplification reaction is a LAMP amplification reaction, the attribute is a fluorescence generated by the reaction. Therefore, the camera captures a fluorescence image of the reaction. The intensity of the fluorescence depends on the presence and/or abundance of the targeted airborne pathogen.

The sensor 211 is preferably located near the microfluidic chip 206, in a location and orientation such that it is able to take pictures/readings from the reactions. The resulting attribute of the reaction (for example fluorescence intensity together with timestamp) is provided to a transceiver 214 which wirelessly sends the attribute to the remote station 215. At the remote station 215 (for example at a cloud server) a prediction of a disease evolution caused by the caught airborne pathogens is performed using the data (attributes) received from the device 219. This process is performed automatically, without requiring human intervention at the device 218 (in general, at the crop field). To achieve this, a control unit 213 monitors the coordinated operation of the components of the device 218.

The control unit 213 is for example a microcontroller that controls operation of the main components of the device 218, such as the means for capturing particles 220, the microfluidic chip 206, the means for releasing DNA 207, the fluid provider 208, the sensor 211 and the transceiver 214. In particular, the control unit 213 is configured to enable that once particles from an air sample have been captured at the means for capturing particles 220, the particles (or free flying pathogens): are provided to the microfluidic chip 206, are impregnated with fluid containing reagents and/or primers and receive microwave radiation. The control unit 213 is also configured to, once a reaction has occurred, to control the sensor 211 so that it captures an attribute of the reaction. And to provide the attribute to the transceiver 214 to be transmitted.

When the device 218 has a fan, the control unit 213 also controls operation of the fan.

Optionally, the device 218 has a weather sensor 212, such as a weather station including weather sensors, configured for measuring weather conditions in the surroundings of the crop field where the device 218 is located. The measured weather conditions are also provided to the transceiver 214, from which they are sent to the remote unit 215. The operation of the weather station 212 is also controlled by the control unit 213. When the device 218 has weather sensors 212, the control unit 213 also controls operation thereof.

Measurements of the weather conditions are required so that the remote unit 215 can perform a prediction with the attributes of the reactions and the weather data captured in the surroundings of the device 218 at the time of obtaining the reactions. Therefore, when a device 218 not having integrated weather sensors 212 is used, weather data must be provided to the remote unit 215 in any other way. For example, there may be weather sensors disposed at the crop field, but independent from the devices 218, configured to send weather data to the remote unit 215. For example, the weather data may be obtained by commercial weather broadcast services providing weather data at the location of the crop field. The remote unit 215 may get the weather data from these services.

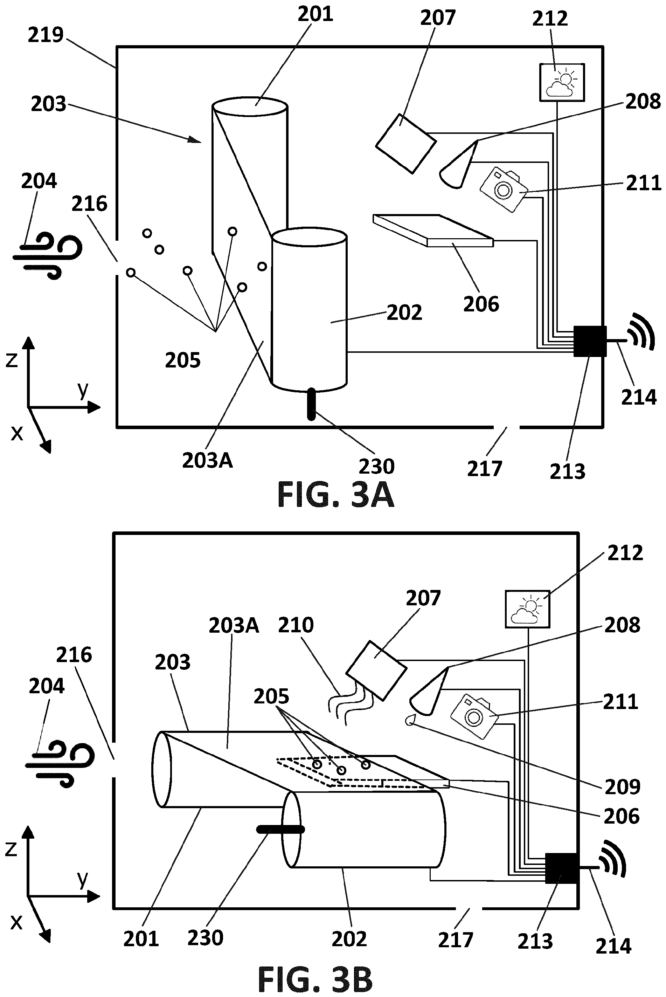

Figure 3A shows a particular device 219 for automatic airborne pathogen detection to be used in a deployment like the one shown in Figure 1. The main difference between the device 218 of Figure 2B and the device 219 of Figure 3A is the means for capturing particles. The remaining components of the device 219 may be similar to the ones shown and disclosed in Figure 2B.

Figure 3A shows a particular embodiment of means for capturing particles 203. The means 203 has an extended portion (spread or unfolded portion) of catching means 203A, such as a portion of strip, tape or paper. This spread portion 203A is the portion of catching means on which particles 205 travelling in the air sample 204 are trapped. The means 203 also has a first spool 201 of catching means and a second spool 202 of catching means. The first spool 201 and second spool 202 may be respective rolls or bobbins of a strip, tape or paper of catching means. The catching means is for example quartz paper.

Initially, the first time the catching means is used, it is completely rolled up in, for example, the first spool 201. A first portion thereof is unrolled and coupled or attached to the other spool (in this case the second spool 202). This is the only stage of the detection process on which human intervention is required, when a new bobbin of catching means is loaded or installed. The unrolled portion is a first portion of exposed portion 203A, which is placed substantially perpendicular to the direction of air flow as air enters the device 219 through the inlet 216. The air sample 204 thus travels through the exposed portion 203A of catching means and airborne pathogens or particles 205 potentially containing airborne pathogens, present in the air sample 204, are caught by the spread portion 203A, trapped thereon. To this end, the means for capturing particles 203 is disposed in a first position, usually a vertical position, in which the exposed portion 203A of catching means is substantially perpendicular to the direction of air flow.

To perform DNA amplification reaction in an automatic way, i.e. without requiring human intervention, the means for capturing particles 203 is rotated from its current position (vertical position) to a rotated position, usually a horizontal position. This is done by rotating means, schematically represented as 230 in Figure 3A. The rotating means 230 may be implemented by a motor and an actuator, not shown. Figure 3B shows the device 219 after this rotation, and therefore in a configuration for performing a DNA amplification reaction of the caught airborne pathogens (or particles containing airborne pathogens) 205. This rotation causes the exposed portion 203A, potentially having caught airborne pathogens, to lie on the microfluidic chip 206, in such a way that the exposed portion 203A overlaps the surface of the microfluidic chip 206. Once in this second rotated position, the dispenser 218, for example located above the exposed portion 203A, delivers the required primers and/or reagents 209, preferably in a fluid solution, towards the exposed portion 203A. At least part of the exposed portion 203A potentially including some caught particles 205 is dissolved and deposited (dragged) by gravity onto the top surface of the microfluidic chip 206, where the DNA amplification reaction can then be performed. After the delivery of primers and/or reagents 209 and subsequent dragging of potential airborne pathogens onto the microfluidic chip 206, the microwave generator 207 located above the microfluidic chip 206 emits microwave radiation 210 in a dosage and intensity suitable for breaking the cell walls of the airborne pathogens, needed for performing the DNA amplification reaction. Depending on the size of the microfluidic chip 206 and on the size of the exposed portion 203A, the microfluidic chip 206 is partially or completely overlapped by the exposed portion 203A, such that the at least some of the captured airborne pathogens, if any, are received by the microfluidic chip.

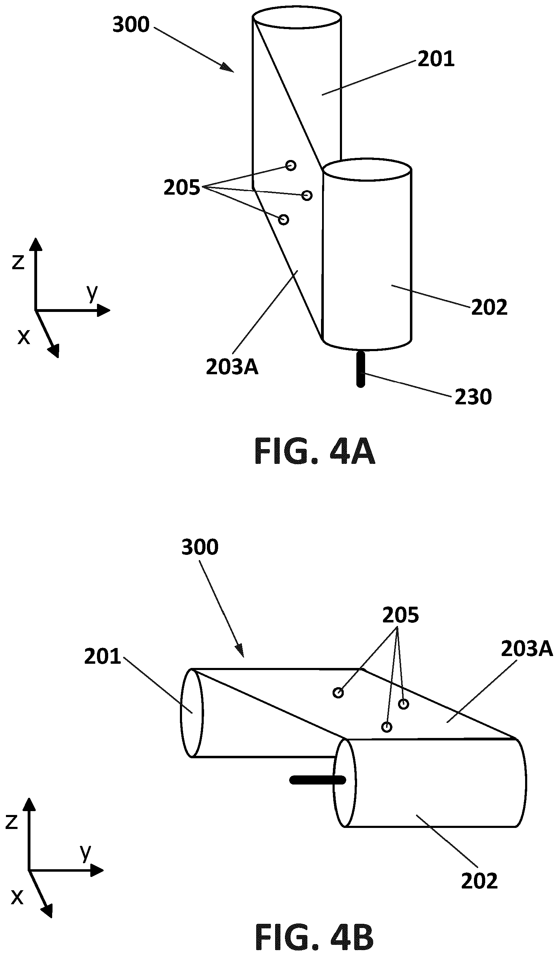

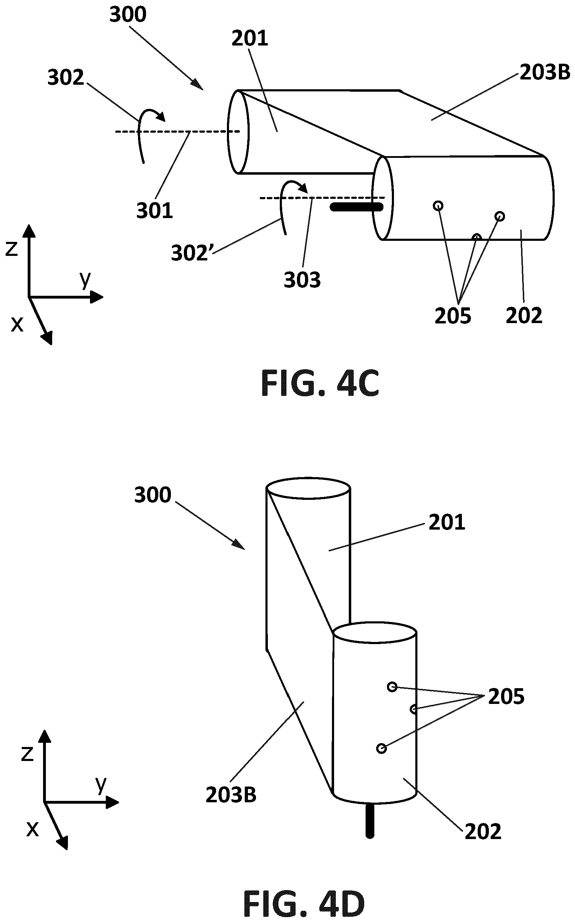

Figures 4A - 4D illustrates the alternate rotation of the means for capturing particles 203 from its first position (vertical position, Figure 4A) to its second position (horizontal position, Figures 4B,4C) and back again to the first position (vertical position, Figure 4D).

In Figures 4A-4B a same exposed portion 203A is shown: In Figure 4A, particles 205 containing airborne pathogens are caught by the exposed part 203A of the catching means. In Figure 4B, after rotating the means for capturing particles 203, the same exposed portion 203A is ready to receive the reagents and/or primers, etc., for which the exposed portion 203A lies above the microfluidic chip (not shown), with which it can be aligned, such that the mix of provided fluid (in general, reagents and/or primers) and particles 205 fall onto the underneath microfluidic chip 206 by gravity.

After a first cycle of particles detection and reaction is performed, a new clean portion of catching means is exposed or unrolled from one of the spools 201, 202 (from the one on which the clean catching means is rolled up). This is shown in Figure 4C. The first spool 201 unrolls a new section of catching means 203B, as shown by arrow 302 around longitudinal axis 301. In a synchronized manner, the second spool 202 rolls-in the used exposed portion 203A, as shown by arrow 302' around longitudinal axis 303.

After unrolling the catching means, such that a new exposed portion 203B is spread, the means for capturing particles 203 is rotated back to its first position (vertical position, Figure 4D), at which a new particle detection cycle starts. The unrolling/rolling of the spools 201, 202 to expose a clean portion of catching means can be performed before rotating back the means for capturing particles 203 to the vertical position. This process continues repeatedly, for example periodically, so that continuous monitoring and capture of airborne pathogens is performed.

The rotation of the means for capturing particles 203 and the rolling up and down of the spools 201, 202 is controlled by the control unit 213 which, applies this control in a coordinated way with the other involved elements. This way, primers and/or reagents are only applied when the exposed means is above the microfluidic chip, microwave radiation is only applied after the primers and/or reagents have been dispensed onto the exposed means, etc.

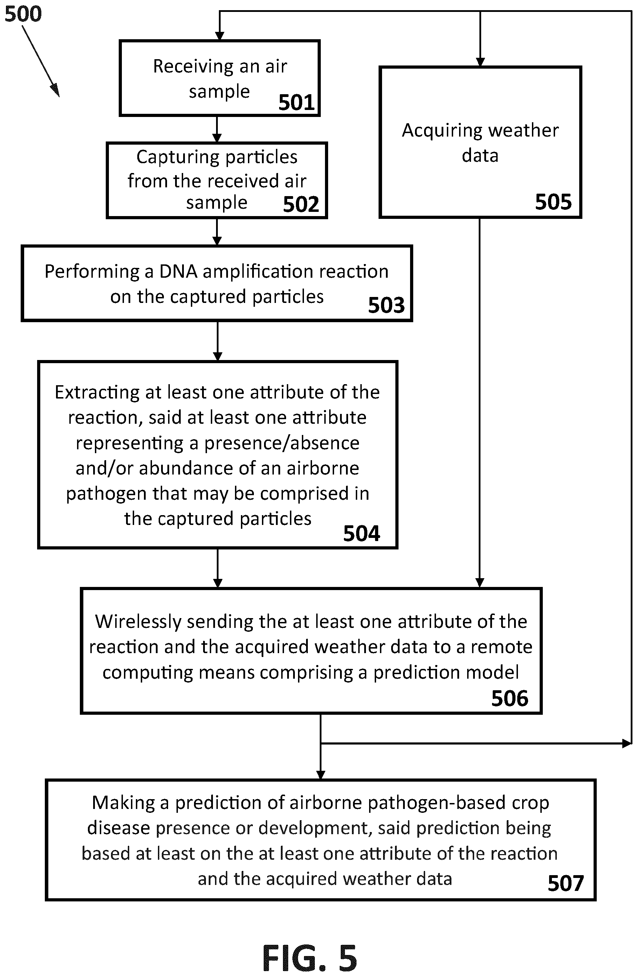

Figure 5 shows a flowchart of the steps performed in a proposed method 500 for predicting diseases in crops. In a first step 501, an air sample from, for example, a crop field, is received. In a second step 502, capturing particles from the received air sample are captured. The particles may comprise airborne pathogens capable of creating a disease in the crops of the crop field. In a third step 503, a DNA amplification reaction on the captured particles is performed. In particular, the DNA amplification reaction is performed on the potential pathogens comprised in the captured particles. In a fourth step 504, at least one attribute of the reaction is extracted. The at least one attribute represents a presence/absence and/or abundance of an airborne pathogen that may be comprised in the captured particles. The former steps are performed in a device 218, 219 like any of the ones already disclosed. In a fifth 505 step, weather data is acquired. The weather data is preferably from the surroundings of the crop field from where the air sample has been received. The weather data can be acquired by sensor(s) integrated within the same device 218, 219. Alternatively, the weather data can be acquired by external sensors, preferably in the vicinity of the device 218, 219. The acquisition of weather data is independent from other steps of the method, such as taking air samples or performing DNA amplification reaction. For example, but not limiting, weather data may be captured by corresponding sensors every 5 minutes, regardless of the frequence of other operations carried out at the device, such as taking air samples or performing DNA amplification reaction. In a sixth step 506, the at least one attribute of the reaction and the acquired weather data are sent to a remote unit, such as a cloud server. In a seventh step 507, a prediction of airborne pathogen-based crop disease presence and/or development is made by the prediction model. The prediction is based at least on the at least one attribute of the reaction and the acquired weather data.

To obtain a suitable prediction model, the prediction model has been previously trained so that it is ready to perform a prediction. To build the model, a machine learning model has been selected and has been duly trained. To train the model, a collection of training data is used. The collection of training data includes: historic weather data captured from the crop field of interest (for example, from the location at which one or more of the devices 218, 219 are deployed) or from locations within a certain distance from the crop field of interest; historic captures of airborne pathonen(s) providing data of the pathogen presence and abundance, typically related to different weather conditions. The weather data has been measured periodically (for example every 5 minutes). The pathogen presence and abundance have been measured on periodic basis (for example daily). The model has been trained with the disease development patterns based on different weather conditions while taking into account pathogen presence and abundance.

In some examples, the training data has been enriched with other data, such as crop phenological stages and/or data about applied crop protection actions.

In this text, the term "comprises" and its derivations (such as "comprising", etc.) should not be understood in an excluding sense, that is, these terms should not be interpreted as excluding the possibility that what is described and defined may include further elements, steps, etc. The term "another," as used herein, is defined as at least a second or more. The term "coupled," when used herein, is defined as connected, whether directly without any intervening elements or indirectly with at least one intervening elements, unless otherwise indicated. Two elements can be coupled mechanically, electrically, or communicatively linked through a communication channel, pathway, network, or system.

The invention is obviously not limited to the specific embodiments described herein, but also encompasses any variations that may be considered by any person skilled in the art (for example, as regards the choice of materials, dimensions, components, configuration, etc.), within the general scope of the invention as defined in the claims.

## Claims

1. A method for predicting diseases in crops, comprising:
a. receiving (501) an air sample (204),
b. capturing (502) particles (205) from the received air sample (204),
c. performing (503) a DNA amplification reaction on the captured particles (205),
d. extracting (504) at least one attribute of the reaction, said at least one attribute representing a presence/absence and/or abundance of an airborne pathogen that may be comprised in the captured particles (205),
e. acquiring (505) weather data,
f. wirelessly sending (506) the at least one attribute of the reaction and the acquired weather data to remote computing means comprising a prediction model,
g. periodically repeating steps a - f, and
h. at the prediction model of the remote computing means, making a prediction (507) of airborne pathogen-based crop disease presence or development,
said prediction being based at least on the at least one attribute of the reaction and the acquired weather data.

2. The method according to claim 1, wherein the acquired weather data comprises information of the following parameters: temperature, relative humidity and precipitation; and optionally of at least one of the following parameters: leaf wetness, wind direction, wind gust and wind speed.

3. The method according to any of the preceding claims, wherein the prediction model is a machine learning model.

4. The method according to claim 3, wherein the machine learning model has been trained using training data comprising: weather data, captures of airborne pathonen(s) and a disease development pattern of potentially captured airborne pathogen(s).

5. The method according to any of the preceding claims, wherein the prediction (507) is a prediction of airborne pathogen-based crop disease development, wherein said prediction predicts a time stage and/or an intensity of the disease development pattern of the captured airborne pathogen.

6. A system (100, 200) for predicting diseases in crops, comprising
at least one device (218, 219) for airborne pathogen detection, the at least one device comprising:
means (216) for receiving an air sample (204),
means (220, 203) for capturing particles (205) from the received air sample (204),
means (206) for performing a DNA amplification reaction on the captured particles (205),
means for extracting at least one attribute of the reaction, said at least one attribute representing a presence/absence and/or abundance of an airborne pathogen that may be comprised in the captured particles (205), and
a transceiver (214) for wirelessly transmitting the at least one extracted attribute of the reaction; and
remote computing means comprising a prediction model, the prediction model being configured to make a prediction of an airborne pathogen-based crop disease presence or development based at least on the at least one extracted attribute of the reaction and on acquired weather data.

7. The system according to claim 6, wherein the acquired weather data comprises information of the following parameters: temperature, relative humidity and precipitation; and optionally of at least one of the following parameters: leaf wetness, wind direction, wind gust and wind speed..

8. The system according to any of claims 6-7, wherein the at least one device (218) further comprises means (207) for breaking a cell wall of the airborne pathogen that may be comprised in the captured particle for liberating DNA of the airborne pathogen prior to the DNA amplification reaction.

9. The system according to any of claims claims 6-8, further comprising means (212) for acquiring the weather data to be used by the prediction model of the remote computing means to make a prediction of an airborne pathogen-based crop disease presence or development, wherein the transceiver (214) of the at least one device (218, 219) is further configured for wirelessly transmitting the acquired weather data together with the at least one extracted attribute of the reaction.

10. The system according to any of claims 6-9, wherein the at least one device further comprises means (208) for providing to the means (220, 203) for capturing particles (205) a reactive element (209) for the DNA amplification reaction.

11. The system according to any of claims 6-10, wherein the means (206) for performing the DNA amplification reaction on the captured particles (205) is a microfluidic chip.

12. A device (219) for automatic airborne pathogen detection, comprising:
• an air sample inlet (216) for allowing entry of an air sample (204) into the device,
• a means for capturing particles (203) configured to, in use of the device, capture particles (205) from the received air sample (204), said means for capturing particles (203) being disposed in a first position,
• rotating means (230) configured to rotate the means for capturing particles (203) to a second position,
• means (208) for providing at least one reactive element (209) to the means for capturing particles (203) when the catching means (203) is in the second position,
• a microfluidic chip (206) configured for performing a DNA amplification reaction on the captured particles (205) and for generating a fluorescence signal based on the amplified DNA of the at least one airborne pathogen that may be comprised in the captured particles (205),
• means (211) for detecting the DNA amplification reaction performed in the microfluidic chip (206),
• means for extracting from the detected DNA amplification at least one attribute of the DNA amplification, said at least one attribute representing the presence/absence and/or abundance of a captured airborne pathogen (205),
• a transceiver (214) configured to wirelessly send said at least one attribute,
and
• a control unit (213) configured to control the rotating means, the means (208) for providing a fluid (209) and the microfluidic chip (206), such that the position of the rotating means is alternatively changed from the first position, to optimize the amount of particles (205) to be captured in the means for capturing particles (203), to the second position, to optimize the provision of fluid (209) to the means for capturing particles (203), and vice versa.

13. The device (218) according to claim 12, wherein the catching means (203) comprises a first spool (201) of particle catching element, a second spool (202) of particle catching element, and a section (203A) of the particle catching element extending between the first spool (201) and the second spool (202), said section (203A) configured for catching airborne particles (205) contained in the air sample (204).

14. The device (218) according to claim 13, wherein the catching means (203) further comprising means for rolling up or down the first spool (201) around a first axis (301) and means for rolling down or up the second spool (202) around a second axis (303), wherein in the means for rolling up or down the first spool (201) and the means for rolling down or up the second spool (202) are configured to roll in a same direction (302), enabling exposure of a new section (203A) of particle catching element to extend between the first spool (201) and the second spool (202).

15. The method according to any of claims 1-5, the system according to any of claims 6-11, or the device according to any of claims 12-14, wherein the airborne pathogens that may be captured are microorganisms, such as fungi, bacteria or viruses.
